(19) Europäisches Patentamt
European Patent Office
Office européen des brevets

(11) **EP 3 653 604 B1**

(12) **EUROPÄISCHE PATENTSCHRIFT**

(45) Veröffentlichungstag und Bekanntmachung des Hinweises auf die Patenterteilung:
**13.10.2021 Patentblatt 2021/41**

(51) Int Cl.:
*C07C 263/10* *(2006.01)* *C07C 265/14* *(2006.01)*

(21) Anmeldenummer: **19208660.1**

(22) Anmeldetag: **12.11.2019**

(54) **VERFAHREN ZUR HERSTELLUNG EINES ISOCYANATS DURCH TEILWEISE ADIABATISCH BETRIEBENE PHOSGENIERUNG DES KORRESPONDIERENDEN AMINS**

METHOD FOR THE PREPARATION OF ISOCYANATE BY PARTIALLY ADIABATIC PHOSGENATION OF THE CORRESPONDING AMINE

PROCÉDÉ DE PRODUCTION D'UN ISOCYANATE PAR PHOSGÉNATION À FONCTIONNEMENT PARTIELLEMENT ADIABATIQUE D'AMINE CORRESPONDANTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2018 EP 18205953**

(43) Veröffentlichungstag der Anmeldung:
**20.05.2020 Patentblatt 2020/21**

(73) Patentinhaber: **Covestro Intellectual Property GmbH & Co. KG**
**51373 Leverkusen (DE)**

(72) Erfinder:
• **KNAUF, Thomas**
**41542 Dormagen (DE)**
• **SPRIEWALD, Jürgen**
**Seabrook, TX 77586 (US)**
• **HIELSCHER, Anke**
**51063 Köln (DE)**
• **MICHELE, Volker**
**51065 Köln (DE)**
• **WASTIAN, Dietmar**
**41542 Dormagen (DE)**
• **STEFFENS, Christian**
**25436 Tornesch (DE)**

(74) Vertreter: **Levpat**
**c/o Covestro AG**
**Gebäude 4825**
**51365 Leverkusen (DE)**

(56) Entgegenhaltungen:
**EP-A1- 1 616 857 WO-A1-2017/001320**

**Beschreibung**

Die vorliegende Erfindung betrifft ein Verfahren zur Herstellung eines Isocyanats durch Umsetzung eines primären Amins mit Phosgen umfassend **I)** Bereitstellung einer Aminlösung, **II)** Bereitstellung einer Phosgenlösung, **III)** Vermischen der Amin- mit der Phosgenlösung in einer Mischeinrichtung gefolgt von **IV)** weiterer Umsetzung in einer adiabatisch betriebenen Reaktionszone und dem Abtrennen der infolge der chemischen Umsetzung gebildeten Gasphase in einer Trennzone, **V)** zwei- bis dreistufiges Entspannen der verbleibenden Flüssigphase, **VI)** weitere Umsetzung der nach der letzten Entspannungsstufe verbleibenden Flüssigphase in einer indirekt beheizten Reaktionszone und **VII)** Isolierung des Isocyanats aus der dort erhaltenen Reaktionslösung.

Isocyanate (1) werden in großen Mengen hergestellt und dienen hauptsächlich als Ausgangsstoffe zur Herstellung von Polyurethanen. Ihre Herstellung erfolgt zumeist durch Umsetzung der entsprechenden Amine (2) mit Phosgen (3), wobei Phosgen im stöchiometrischen Überschuss eingesetzt wird. Die Umsetzung der Amine mit dem Phosgen kann sowohl in der Gasphase als auch in der Flüssigphase erfolgen, wobei die Reaktion diskontinuierlich oder kontinuierlich durchgeführt werden kann. Die Phosgenierungsreaktion liefert - im Fall der Gasphasenphosgenierung nach der sog. *Quenche* des zunächst anfallenden gasförmigen Reaktionsprodukts - eine Flüssigphase enthaltend das gewünschte Isocyanat. Neben dieser Flüssigphase fallen an verschiedenen Stellen des Prozesses Gasströme an, die, nachdem diese soweit technisch möglich und wirtschaftlich sinnvoll von Wertprodukten wie Chlorwasserstoff, Phosgen, Isocyanat und Lösungsmittel befreit wurden, im Allgemeinen einer Phosgenzersetzung zugeführt werden, in welcher Spurenanteile an Phosgen, die in den vorangegangenen Aufarbeitungsschritten nicht abgetrennt werden konnten, katalytisch mit Wasser zersetzt werden. Im Allgemeinen wird hierfür Aktivkohle als Katalysator eingesetzt. Diese Phosgenzersetzung liefert ein gereinigtes Abgas und einen sauren Abwasserstrom, der entsorgt werden muss. Dieser Abwasserstrom enthält im Allgemeinen noch organische Verunreinigungen wie beispielsweise Lösungsmittel (im Fall der Herstellung von MDI üblicherweise Monochlorbenzol), Amin (im Fall der Herstellung von MDI Anilin) und Harnstoffverbindungen. Diese organischen Verunreinigungen müssen weitestgehend entfernt werden, bevor das Abwasser einer Abwasseraufbereitungsanlage (zum Beispiel einer biologischen Kläranlage) zugeführt werden kann. Eine Möglichkeit, dies zu erreichen, besteht darin, dass Abwasser durch Zugabe von Base (zum Beispiele Natronlauge) auf einen pH-Wert > 7, insbesondere im Bereich von 11 bis 13, zu bringen und im Anschluss die organischen Verunreinigungen an Aktivkohle zu adsorbieren. Eine solche Adsorption an Aktivkohle ermöglicht auf einfache Weise die Reduktion der Konzentration an organischen Verunreinigungen in diesem Abwasserstrom auf ein Niveau, das es erlaubt, das Abwasser einer Abwasseraufbereitungsanlage zuzuführen.

Verfahren zur Herstellung von organischen Isocyanaten aus primären Aminen und Phosgen sind bereits vielfach beschrieben worden; rein exemplarisch sei auf die folgenden Schriften verwiesen:
DE-A-34 03 204 beschreibt ein kontinuierliches Verfahren zur Herstellung organischer Polyisoyanate, bei welchem bei einem Druck von 5 bis100 bar in einer zum Teil im Kreislauf geführten Reaktion erhöhte Temperaturen von 100 bis 220 °C eingestellt werden.

DE-A-17 68 439 beschreibt ein Verfahren zur fortlaufenden Herstellung organischer Isocyanate, bei welchem die Einsatzstoffe Amin und Phosgen zunächst vorerhitzt und dann die vorerhitzten Bestandteile in der Reaktionszone unter hohen Drücken zusammengeführt und unter isothermen Bedingungen, d. h. unter Wärmeaustausch mit der Umgebung, zur Reaktion gebracht werden.

DE-A-102 22 968 beschreibt ein Verfahren zur kontinuierlichen Herstellung von Polyisocyanaten durch Umsetzung von primären Aminen mit Phosgen, bei welchem die Umsetzung in einer Kaskade von temperierbaren Reaktionsrohren unterschiedlicher Größe durchgeführt wird.

EP 1 873 142 A1 beschreibt eine dreistufige Verfahrensführung, bei welcher der Druck zwischen der ersten Stufe eines Mischers und der zweiten Stufe eines ersten Phosgenierreaktors gleichbleibt oder ansteigt und in der dritten Stufe, einem Apparat zur Phosgenabtrennung, der Druck niedriger ist als in der zweiten Stufe. Die Reaktion kann adiabatisch oder isotherm betrieben werden.

Von großtechnischem Interesse sind sowohl aromatische Isocyanate wie beispielsweise Methylen-Diphenylen-Diisocyanat (fortan MMDI - "monomeres MDI"), Gemische aus MMDI und Polymethylen-Polyphenylen-Polyisocyanate (das sind die höheren Homologen des MMDI, fortan PMDI, "polymeres MDI") oder Toluylendiisocyanat (TDI) als auch aliphatische Isocyanate wie z. B. 1,5-Pentandiisocyanat (PDI), 1,6-Hexamethylendiisocyanat (HDI), oder Isophorondiisocyanat (IPDI). Daneben sind auch Isocyanate mit benzylischen Isocyanatgruppen bedeutsam, insbesondere sei hier Xylylendiisocyanat (XDI) erwähnt. Die vorliegende Erfindung befasst sich insbesondere mit der Herstellung von Methylen-Diphenylen-Diisocyanaten und Polymethylen-Polyphenylen-Polyisocyanaten (fortan summarisch MDI genannt).

Bei der Mehrzahl der bekannten Verfahren werden zur Einstellung der gewünschten Reaktionstemperatur temperierbare Reaktoren in unterschiedlichen Varianten (Mantelheizung, Beheizung durch Wärmeaustauscher oder spezielle Reaktoreinbauten) eingesetzt. Bei der Isocyanatsynthese durch Phosgenierung von Aminen stellt die externe Temperierung der Reaktoren jedoch oft ein Problem dar, da die hohen Temperaturen der Reaktorwandflächen die Bildung von Nebenprodukten fördern oder sogar erst verursachen, welche dann die Ausbeute und/oder Produkteigenschaften nachteilig beeinflussen. Außerdem werden dann im Reaktor Ablagerungen gebildet, die ein regelmäßiges Abfahren und Reinigen der Reaktoren erforderlich machen. Dies führt aber zum Verlust von Anlagenkapazität und somit zu einem wirtschaftlichen Nachteil. Darüber hinaus verursachen die Wärmeträgeranlagen zusätzliche Investitionskosten, was die Wirtschaftlichkeit des Verfahrens ebenfalls verschlechtert. Zur Lösung dieser Probleme wird in EP 1 616 857 A1 eine zweistufige Verfahrensführung vorgeschlagen, bei welcher in einer ersten Stufe a) Amin und Phosgen in einer *adiabatisch geführten Reaktion* zur Umsetzung gebracht werden, wobei die Temperatur der Umsetzung auf Werte zwischen 100 und 220 °C begrenzt wird, indem der absolute Druck im Reaktor durch Entspannung gezielt auf Werte zwischen 8 und 50 bar eingestellt wird, und die Temperatur bei Werten zwischen 100 und 220 °C so lange beibehalten wird, bis ein Umsatz an Phosgen von mindestens 80 % erreicht ist, und anschließend in einer zweiten Stufe b) das Reaktionsgemisch aus der ersten Stufe auf absolute Drücke im Bereich von 1 bis 15 bar entspannt und bei Temperaturen zwischen 90 und 240°C, *üblicherweise unter Zufuhr von Wärme,* weiter umgesetzt wird. Eine solche Verfahrensführung kann als *adiabatisch-isotherme Verfahrensführung* bezeichnet werden. Wesentlich für das beschriebene Verfahren ist die Einstellung der Temperatur der Umsetzung im adiabatisch betriebenen Reaktor (100 °C bis 220 °C, bevorzugt 115 °C bis 180 °C, besonders bevorzugt 120 °C bis 150 °C) durch den Druck in diesem Reaktor. Diese Einstellung durch den Druck erfolgt durch kontrolliertes Entspannen mittels Öffnens von an dem Reaktor angebrachten Ventilen, wobei Teile des Reaktionsgemisches aus dem Reaktor entweichen (vgl. Absatz [0016]). Das den adiabatisch betriebenen Reaktor verlassende Reaktionsgemisch wird in einer zweiten Stufe unter isothermen Bedingungen weiter umgesetzt und dabei auf einen Druck unterhalb dem der ersten Stufe entspannt (vgl. Absatz [0019]). Am Ausgang des isotherm betriebenen Reaktors werden diesem eine Gasphase und eine das Isocyanat enthaltende Flüssigphase separat entnommen.

Die internationale Patentanmeldung WO2017/001320 A1 befasst sich mit einem Verfahren zur effizienten Bereitstellung des in der Phosgenierung von Aminen als Koppelprodukt anfallenden Chlorwasserstoffs für Folgeanwendungen (wie beispielsweise einen *Deacon*-Prozess). In diesem Verfahren wird das Rohprodukt *der Phosgenierung (also das nach maximalem Reaktionsfortschritt erhaltene Verfahrensprodukt),* gegebenenfalls nach Abtrennung eines überwiegend Chlorwasserstoff enthaltenden Purge-Stromes noch während der Phosgenierung selbst, ein- oder mehrstufig auf einen Druck $p_E$, der *größer* ist als der für die Folgeanwendung erforderliche Druck $p_F$, entspannt, und die verbleibende, das angestrebte Isocyanat enthaltende Flüssigphase wird einer an sich bekannten Entphosgenierung unterworfen. Der bei der Entphosgenierung erhaltene, Phosgen- und Chlorwasserstoff-haltige Gasstrom weist dabei einen Druck auf, der *geringer* ist als der für die Folgeanwendung erforderliche Druck. Dieser letztgenannte Gasstrom wird daher verdichtet, mit dem in der Entphosgenierung erhaltenen Gasstrom vereint und anschließend einer an sich bekannten HCl-Phosgen-Trennung unterzogen, welche einen gereinigten Chlorwasserstrom ergibt, der unter einem solchen Druck steht, dass er *ohne Verdichtung* der ihm zugedachten Folgeanwendung zugeführt werden kann (vgl. FIG. 1 und die zugehörigen Erläuterungen in der Beschreibung). Der Vorteil dieser Vorgehensweise besteht darin, dass ein beträchtlicher Teil des insgesamt anfallenden Chlorwasserstoffs, nämlich der in der ein- oder mehrstufigen Entspannung des Rohprodukts anfallende Teil, ohne Verdichtung der HCl-Phosgen-Trennung und der Folgeanwendung zugeführt werden kann, was den Bau kleinerer Kompressoren ermöglicht. Das Verfahren beruht letztlich darauf, dass die Reaktion bei vergleichsweise hohen Drücken durchgeführt wird, was es ermöglicht, die Entspannung des Rohprodukts der Phosgenierung auf ein immer noch vergleichsweise hohes Druckniveau durchzuführen. Die Entspannung erfolgt dabei insbesondere ausgehend von Drücken in der Reaktion im Bereich von 6,00 bar bis 60,0 bar auf Drücke der der Entspannung entnommenen Chlorwasserstoff-haltigen Gasphase im Bereich von 5,00 bar bis 30,0 bar, bevorzugt ausgehend von Drücken in der Reaktion im Bereich von 12,0 bar bis 45,0 bar auf Drücke der der Entspannung entnommenen Chlorwasserstoff-haltigen Gasphase im Bereich von 9,00 bar bis 18,0 bar. Im Falle einer mehrstufigen Entspannung offenbart die Schrift den Einsatz einer Kaskade mehrerer hintereinandergeschalteter Abscheider mit sukzessive sinkendem Druckniveau, wobei die in den einzelnen Abscheidern jeweils erhaltenen Gasphasen zum Gasstrom mit dem Druck $p_E$ vereinigt werden, was augenscheinlich bedeutet, dass alle Gasphasen auf den Druck der im letzten Abscheider erhaltenen Gasphase entspannt werden.

Die der ein- oder mehrstufigen Entspannung *vorgelagerte* Umsetzung des Amins mit Phosgen kann dabei gemäß der Lehre der WO 2017/001320 A1 auch in einer Sequenz aus einem adiabatisch und einem isotherm betriebenen Reaktor durchgeführt werden wie in EP 1 616 857 A1 beschrieben. Bedenkt man, dass der Druck in der isothermen Stufe des Verfahrens gemäß EP 1 616 857 A1 immer noch bis zu 15 bar betragen kann, wird dies verständlich. Eine Verbindung der Lehre der WO 2017/001320 A1 mit der Lehre der EP 1 616 857 A1 würde den Fachmann daher dazu veranlassen, das in der WO 2017/001320 A1 beschriebene Verfahren *nach* der in der EP 1 616 857 A1 beschriebenen isothermen Stufe (also *nach* maximalem Reaktionsfortschritt) anzuwenden. Eine Sequenz aus (**a**) adiabatischer Reaktionsführung, (**b**) Trennung von dort gebildeter Gas- und Flüssigphase, (**c**) mehrstufige Entspannung der abgetrennten Flüssigphase und (**d**) weitere Umsetzung derselben unter isothermen Bedingungen wird in der Anmeldung WO 2017/001320 A1 daher nicht offenbart.

Die Güte eines Verfahrens zur Herstellung von Isocyanaten ist einerseits definiert durch den Gehalt an unerwünschten Nebenprodukten im Produkt des Verfahrens. Andererseits ist die Güte eines Verfahrens dadurch definiert, dass der gesamte Prozess von Anfahren, Produktion im Regelbetrieb bis zum Abfahren des Prozesses ohne technischen Produktionsausfall und ohne Probleme, die zu einem Eingriff in den Prozess nötigen würden, betrieben werden kann, und dass es nicht zu Verlusten an Einsatzstoffen, Zwischenprodukten oder an Endprodukt kommt.

Idealerweise sind daher die großtechnischen Anlagen zur Durchführung solcher Herstellungsverfahren derart ausgelegt, dass bei einer passenden Qualität der eingesetzten Hilfsund Einsatzstoffe und richtiger Wahl der Verfahrensparameter wie Druck, Temperatur, Mengenverhältnisse und Konzentrationen der Hilfs- und Einsatzstoffe etc. die Verfahren robust verlaufen. Das bedeutet, dass es in solchen kontinuierlich betriebenen Großanlagen idealerweise nicht zu Problemen wie etwa der Entstehung von Niederschlägen kommt, die sich an Anlagenteilen absetzen und beispielsweise Rohrleitungen verstopfen können. Andererseits trägt auch eine optimale Ausnutzung der Raum-Zeit-Ausbeute in einem nicht unerheblichen Umfang zu einer Verbesserung der Produktivität und somit der Wirtschaftlichkeit von großtechnischen Phosgenieranlagen bei. Geht man bei der Produktionsrate in die Grenzbereiche des noch Machbaren einer gegebenen Anlage, dann müssen die Verfahrensparameter in einem sehr engen Fenster betrieben werden, damit es nicht zu den oben geschilderten Problemen mit Niederschlägen, Anbackungen und Produktqualität kommt.

Bei der prinzipiell bekannten (siehe EP 1 616 857 A1) zweistufigen adiabatisch-isothermen Verfahrensführung mit *deutlich unterschiedlichen* Drücken in jeder Stufe (siehe die Beispiele dieser Anmeldung: 22 bar in der ersten Stufe und 2 bar in der zweiten Stufe) kann es bei der Druckerniedrigung nach der adiabatisch betriebenen Stufe zu einem Aufschäumen der Reaktionslösung, unter Umständen unter Feststoffbildung, kommen. Ferner muss bei allen Betriebsbedingungen die Funktionalität der Regelung (Druckhaltung des Gases und der Flüssigkeit) im Schritt der Druckerniedrigung gewährleistet sein. Schließlich ist ausreichend Verweilzeit zur Verfügung zu stellen, um die saubere Abtrennung der bei der Druckerniedrigung gebildeten Gasphase von der verbleibenden Flüssigphase sicherzustellen, ohne die Apparatedimensionen (und damit insbesondere die Investitionskosten) allzu groß werden zu lassen.

Zusammenfassend kann daher festgestellt werden, dass in einer zweistufigen adiabatisch-isothermen Verfahrensführung mit deutlich unterschiedlichen Drücken in den beiden Stufen der Schritt der Druckerniedrigung unter verfahrens- und regelungstechnischen Gesichtspunkten sowie im Hinblick auf eine möglichst große Wirtschaftlichkeit des Verfahrens nicht ohne Herausforderungen ist und hier noch Verbesserungsbedarf gegenüber dem Stand der Technik bestand.

Diesem Bedarf Rechnung tragend ist ein Gegenstand der vorliegenden Erfindung ein **Verfahren zur Herstellung eines Isocyanats durch Umsetzung eines primären Amins mit Phosgen,** umfassend die Schritte:

I) Bereitstellen einer Lösung des primären Amins in einem Lösungsmittel;

II) Bereitstellen einer Lösung von Phosgen in einem Lösungsmittel;

III) Vermischen der in Schritt I) bereitgestellten Lösung des primären Amins und der in Schritt II) bereitgestellten Lösung von Phosgen in einer Mischeinrichtung zu einem Reaktionsgemisch einer Temperatur im Bereich von 110 °C bis 145 °C unter Einhaltung eines auf die Aminogruppen des primären Amins bezogenen stöchiometrischen Überschusses an Phosgen im Bereich von 40 % bis 200 % der Theorie, bevorzugt im Bereich von 40 % bis 120 % der Theorie, besonders bevorzugt im Bereich von 50 % bis 100 % der Theorie, ganz besonders bevorzugt im Bereich von 50 % bis 75 % der Theorie;

IV) Führen des in Schritt III) erhaltenen flüssigen Reaktionsgemisches durch eine Reaktionszone und durch eine dieser Reaktionszone strömungstechnisch nachgeschaltete Trennzone unter Ausbildung einer unter einem Druck im Bereich von 8,0 $bar_{(abs.)}$ bis 50,0 $bar_{(abs.)}$, insbesondere im Bereich von 15,0 $bar_{(abs.)}$ bis 30,0 $bar_{(abs.)}$, stehenden Gasphase aus dem flüssigen Reaktionsgemisch in der Trennzone, wobei die Reaktionszone und die Trennzone nicht beheizt und nicht gekühlt werden, wobei die in der Trennzone gebildete Gasphase und die verbleibende Flüssigphase der Trennzone getrennt voneinander entnommen werden;

V) Entspannen der der Trennzone aus Schritt IV) entnommenen Flüssigphase unter partieller Überführung dieser Flüssigphase in die Gasphase;

VI) Führen der in Schritt V) nach dem Entspannen verbleibenden Flüssigphase durch eine indirekt beheizte Reaktionszone, wobei sich eine Chlorwasserstoff- und Phosgen-haltige Gasphase (die im Allgemeinen auch noch Anteile verdampften Lösungsmittels enthält) ausbildet, die abgetrennt wird, und eine Isocyanat- und Lösungsmittel enthaltende Flüssigphase verbleibt, die der indirekt beheizten Reaktionszone entnommen wird;

VII) Aufarbeiten der in Schritt VI) gewonnenen Isocyanat- und Lösungsmittel enthaltenden Flüssigphase unter Rückgewinnung des Lösungsmittels und Erhalt des Isocyanats;

wobei in Schritt V) zum Entspannen der der Trennzone aus Schritt IV) entnommenen Flüssigphase diese Flüssigphase

(i) zunächst in einem ersten Gas-Flüssigkeits-Trennbehälter entspannt wird, wobei eine erste Flüssigphase und eine erste Gasphase gebildet werden,
(ii) woran anschließend die erste Flüssigphase in einem zweiten Gas-Flüssigkeits-Trennbehälter weiter entspannt wird, wobei eine zweite Flüssigphase und eine zweite Gasphase gebildet werden,
(iii) woran anschließend optional die zweite Flüssigphase in einem dritten Gas-Flüssigkeits-Trennbehälter weiter entspannt wird, wobei eine dritte Flüssigphase und eine dritte Gasphase gebildet werden,

wobei die in Schritt VI) durch die indirekt beheizte Reaktionszone geführte Flüssigphase die zweite (wenn Schritt (iii) nicht umfasst ist) oder (wenn Schritt (iii) umfasst ist) die dritte Flüssigphase ist.

Erfindungsgemäß steht die sich in der Trennzone ausbildende Gasphase *"unter einem Druck im Bereich von 8,0 bar$_{(abs.)}$ bis 50,0 bar$_{(abs.)}$, insbesondere im Bereich von 15,0 bar$_{(abs.)}$ bis 30,0 bar$_{(abs.)}$"*. Durch die in der Reaktionszone ablaufenden chemischen Umsetzungen bildet sich aus dem in Schritt (III) erhaltenen flüssigen Reaktionsgemisch eine (mindestens) Chlorwasserstoffund Phosgen-haltige Gasphase. Die genannten Druckwerte beziehen sich also auf den Gasraum der Trennzone. Hier und im Folgenden sind alle Drücke als absolute Drücke (gekennzeichnet als "bar$_{(abs.)}$") zu verstehen.

Da die Trennzone der Reaktionszone *"strömungstechnisch nachgeschaltet"* ist, womit in der Terminologie der vorliegenden Erfindung auch eine offene, strömungstechnische Verbindung beider Zonen gemeint ist, und da ferner die Reaktions- und Trennzone *"nicht beheizt und nicht gekühlt werden"* (= adiabatische Verfahrensführung), stellt sich an jeder Stelle der Reaktions- und Trennzone eine Temperatur ein, die bei gegebener Temperatur des Reaktionsgemisches aus Schritt (III) im Wesentlichen - abgesehen von Wärmeverlusten durch nicht perfekte Isolierung der verwendeten Apparaturen - von den Reaktionsenthalpien der ablaufenden chemischen Prozesse (die weiter unten noch im Detail erläutert werden) bestimmt wird. Auch der sich etablierende Druck wird zunächst von den ablaufenden chemischen Prozessen bestimmt. Vorzugsweise ist jedoch in der Trennzone ein Druckhalteventil für die sich ausbildende Gasphase und eine Flüssigkeitsstandregelung für die Flüssigphase vorgesehen, um sicher gewährleisten zu können, dass Druck im oben genannten Bereich - 8,0 bar$_{(abs.)}$ bis 50,0 bar$_{(abs.)}$, insbesondere im Bereich von 15,0 bar$_{(abs.)}$ bis 30,0 bar$_{(abs.)}$ - liegt. Somit hängt aber die Temperatur in Schritt IV) letztlich von der Temperatur des diesem Schritt zuzuführenden Reaktionsgemisches ab.

Erfindungsgemäß wird Phosgen, bezogen auf die Aminogruppen des primären Amins, im *"stöchiometrischen Überschuss"* eingesetzt. Theoretisch reagiert 1 Mol Phosgen mit 1 Mol primärer Aminogruppen (1 R-NH$_2$ + 1 COCl$_2$ → 1 R-NCO + 2 HCl). Ein Phosgenüberschuss von x % gegenüber primären Aminogruppen entspricht daher einem molaren Verhältnis $n$(Phosgen)/$n$(-NH$_2$) ($n$ = Stoffmenge) von $\dfrac{1 + \frac{x}{100}}{1}$, also beispielsweise $\dfrac{1 + \frac{40}{100}}{1} = 1{,}40$ bei 40%igem Phosgenüberschuss oder beispielsweise $\dfrac{1 + \frac{120}{100}}{1} = 2{,}2$ bei 120%igem Phosgenüberschuss.

Es folgt zunächst eine <u>Kurzzusammenfassung</u> verschiedener möglicher **Ausführungsformen der Erfindung**:

In einer **ersten Ausführungsform der Erfindung**, die mit allen anderen Ausführungsformen kombiniert werden kann, weist die in Schritt I) bereitgestellte Lösung des primären Amins einen auf die Gesamtmasse dieser Lösung bezogenen Massenanteil an primärem Amin im Bereich von 25 % bis 50 %, insbesondere im Bereich von 30 % bis 45 %, auf und die in Schritt II) bereitgestellte Lösung von Phosgen weist einen auf die Gesamtmasse dieser Lösung bezogenen Massenanteil an Phosgen im Bereich von 45 % bis 90 %, insbesondere im Bereich von 55 % bis 80 %, auf.

In einer **zweiten Ausführungsform der Erfindung**, die mit allen anderen Ausführungsformen kombiniert werden kann, wird die Mischeinrichtung aus Schritt III) nicht beheizt und nicht gekühlt.

In einer **dritten Ausführungsform der Erfindung**, die mit allen anderen Ausführungsformen kombiniert werden kann, umfasst die in Schritt III) eingesetzte Mischeinrichtung einen oder mehrere dynamische Mischer und umfasst insbesondere keinen statischen Mischer.

In einer **vierten Ausführungsform der Erfindung**, die mit allen anderen Ausführungsformen kombiniert werden kann, sind die Reaktionszone und Trennzone aus Schritt IV) in einem gemeinsamen Reaktor angeordnet.

In einer **fünften Ausführungsform der Erfindung**, die eine besondere Ausgestaltung der vierten Ausführungsform ist, wird als Reaktor ein aufrecht angeordneter röhrenförmiger Reaktor eingesetzt.

In einer **sechsten Ausführungsform der Erfindung**, die eine besondere Ausgestaltung der fünften Ausführungsform ist, wird der Reaktor mit dem in Schritt III) erhaltenen Reaktionsgemisch von unten nach oben durchströmt.

In einer **siebten Ausführungsform der Erfindung**, die mit allen anderen Ausführungsformen kombiniert werden kann, ist die indirekt beheizte Reaktionszone aus Schritt VI) Bestandteil eines Rohrbündelreaktors, durch dessen Rohrinnenraum die in Schritt V) nach dem Entspannen verbleibende Flüssigphase geführt wird und durch dessen Rohraußenraum ein Heizmedium geführt wird, oder durch dessen Rohraußenraum die in Schritt V) nach dem Entspannen verbleibende Flüssigphase geführt wird und durch dessen Rohrinnenraum ein Heizmedium geführt wird.

In einer **achten Ausführungsform der Erfindung,** die mit allen anderen Ausführungsformen kombiniert werden kann, ist die indirekt beheizte Reaktionszone aus Schritt VI) Bestandteil eines Rohrbündelreaktors, umfasst das Entspannen in Schritt V) nur die Stufen (i) und (ii), wobei das Entspannen so durchgeführt wird, dass die erste Gasphase unter einem Druck im Bereich von 10 bar$_{(abs.)}$ bis 20 bar$_{(abs.)}$, insbesondere 12 bar$_{(abs.)}$ bis 17 bar$_{(abs.)}$, anfällt, und die zweite Gasphase unter einem Druck im Bereich von 1,0 bar$_{(abs.)}$ bis 5,0 *bar*$_{(abs.)}$, insbesondere 2,0 bar$_{(abs.)}$ bis 3,0 bar$_{(abs.)}$, anfällt.

In einer **neunten Ausführungsform der Erfindung,** die mit allen Ausführungsformen, die nicht auf zwei Entspannungsstufen beschränkt sind, kombiniert werden kann, umfasst das Entspannen in Schritt V) die Stufen (i), (ii) und (iii), wobei das Entspannen so durchgeführt wird, dass die erste Gasphase unter einem Druck im Bereich von 15 bar$_{(abs.)}$ bis 20 bar$_{(abs.)}$, insbesondere 17 bar$_{(abs.)}$ bis 18 bar$_{(abs.)}$, anfällt, und die zweite Gasphase unter einem Druck im Bereich von 5,0 bar$_{(abs.)}$ bis 10 *bar*$_{(abs.)}$, insbesondere 7,0 bar$_{(abs.)}$ bis 8,0 bar$_{(abs.)}$, anfällt, und die dritte Gasphase unter einem Druck im Bereich von 1,0 bar$_{(abs.)}$ bis 5,0 bar$_{(abs.)}$, insbesondere 2,0 bar$_{(abs.)}$ bis 3,0 bar$_{(abs.)}$, anfällt.

In einer **zehnten Ausführungsform der Erfindung,** die mit allen anderen Ausführungsformen kombiniert werden kann, werden die in den Schritten IV), V) und VI) anfallenden Gasphasen zur Gewinnung von Chlorwasserstoff und Phosgen sowie gegebenenfalls Lösungsmittel aufgearbeitet.

In einer **elften Ausführungsform der Erfindung,** die eine besondere Ausgestaltung der zehnten Ausführungsform ist, werden die in den Schritten IV), V) und VI) anfallenden Gasphasen vor der Aufarbeitung auf einen gemeinsamen Druck eingestellt und vereinigt.

In einer **zwölften Ausführungsform der Erfindung,** die eine besondere Ausgestaltung der elften Ausführungsform ist, werden die *in Schritt IV)* erhaltene Gasphase, die in Schritt V) erhaltene *zweite* Gasphase und die gegebenenfalls in diesem Schritt erhaltene *dritte* Gasphase, sowie die *in Schritt VI)* erhaltene Gasphase auf den Druck der *ersten* in Schritt V) erhaltenen Gasphase eingestellt.

In einer **dreizehnten Ausführungsform der Erfindung,** die eine besondere Ausgestaltung der zwölften Ausführungsform ist, werden die vereinigten Gasphasen kondensiert und komprimiert und zur Trennung von Chlorwasserstoff und Phosgen destilliert.

In einer **vierzehnten Ausführungsform der Erfindung,** die mit allen anderen Ausführungsformen kombiniert werden kann, wird

(i) Methylen-Diphenylen-Diisocyanat und/oder Polymethylen-Polyphenylen-Polyisocyanat durch Umsetzung von Methylen-Diphenylen-Diamin und/oder Polymethylen-Polyphenylen-Polyamin mit Phosgen oder

(ii) Toluylendiisocyanat durch Umsetzung von Toluylendiamin mit Phosgen hergestellt.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

**Schritt I)** der vorliegenden Erfindung, die Bereitstellung der für die Phosgenierung erforderlichen Aminlösung, kann nach allen aus dem Stand der Technik bekannten Verfahren erfolgen. Das einzusetzende Amin wird durch das angestrebte Isocyanat bestimmt. Das erfindungsgemäße Verfahren ist grundsätzlich zur Herstellung beliebiger aromatischer, aliphatischer und araliphatischer Isocyanate geeignet. Bevorzugt eingesetzt wird das erfindungsgemäße Verfahren zur Herstellung von Methylen-Ddiphenylen-Diisocynat (aus Methylen-Diphenylen-Diamin), Polymethylen-Polyphenylen-Polyisocyanat (aus Polymethylen-Polyphenylen-Polyamin), Gemischen aus Methylen-Diphenylen-Diisocyanat und Polymethylen-Polyphenylen-Polyisocyanat (diese Gemische werden fortan auch als MDI und die Ausgangsamin-Gemische als MDA bezeichnet), Toluylendiisocyanat (aus Toluylendiamin), Xylylendiisocyanat (aus Xylylendiamin), 1,5-Pentandiisocyanat (aus 1,5-Pentandiamin), Hexamethylendiisocyanat (aus Hexamethylendiamin), Isophorondiisocyanat (aus Isophorondiamin) und Naphthyldiisocyanat (aus Naphthyldiamin), besonders bevorzugt von Methylen-Diphenylen-Diisocyanat, Gemischen aus Methylen-Diphenylen-Diisocyanat und Polymethylen-Polyphenylen-Polyisocyanat sowie Toluylendiisocyanat. Ganz besonders bevorzugt eignet sich das erfindungsgemäße Verfahren zur Herstellung von Methylen-Diphenylen-Diisocyanat und Gemischen aus Methylen-Diphenylen-Diisocyanat und Polymethylen-Polyphenylen-Polyisocyanat. Methylen-Diphenylen-Diisocyanat wird auch als Diisocyanat der Diphenylmethanreihe bezeichnet. Polymethylen-Polyphenylen-Polyisocyanat wird auch als Polisocvanat der Diphenylmethanreihe bezeichnet.

Verfahren zur Herstellung der genannten Amine sind dem Fachmann bekannt und bedürfen daher keine weitere Erläuterung an dieser Stelle.

In Schritt I) wird das zu phosgenierende Amin in einem Lösungsmittel gelöst. Dies kann mittels dem Fachmann bekannter Mischeinrichtungen wie insbesondere Mischrohren mit statischen Mischern als Einbauten (häufig auch kurz als *Statikmischer* bezeichnet) geschehen. Geeignete erfindungsgemäß einsetzbare *Lösungsmittel* sind dabei unter den Reaktionsbedingungen inerte Lösungsmittel wie z. B. Monochlorbenzol, Dichlorbenzol (insbesondere das *ortho*-Isomer), Dioxan, Toluol, Xylol, Methylenchlorid, Perchlorethylen, Trichlorfluormethan oder Butylacetat. Das Lösungsmittel ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von Recycleströmen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Monochlorbenzol (MCB) oder *ortho*-Dichlorbenzol (ODB) eingesetzt, ganz besonders bevorzugt Monochlorbenzol (MCB).

Bevorzugt ist eine Temperatur der resultierenden Amin-Lösung im Bereich von 30 °C bis 130 °C, insbesondere im Bereich von 60 °C bis 100 °C. Dies kann prinzipiell erreicht werden durch entsprechende Temperierung der Ausgangsstoffe Amin und Lösungsmittel unter Berücksichtigung der Lösungsenthalpie. Es ist erfindungsgemäß jedoch bevorzugt, insbesondere zusätzlich zur genannten Temperierung der Ausgangsstoffe, einen der Vermischung von Amin und Lösungsmittel nachgeschalteten Wärmeaustauscher bereitzustellen, der die gezielte Einstellung der Amin-Lösung auf die gewünschte Temperatur im Bereich von 30 °C bis 130 °C, insbesondere im Bereich von 60 °C bis 100 °C, ermöglicht, der also je nachdem welche Temperatur unmittelbar nach der Vermischung der Ausgangsstoffe vorliegt, heizen oder kühlen kann. Hierzu eignen sich dem Fachmann bekannte Wärmeaustauscher wie insbesondere Rohrbündelwärmeaustauscher und Plattenwärmeaustauscher.

Im Hinblick auf die Aminkonzentration in der in Schritt I) bereitgestellten Lösung ist es bevorzugt, den auf die Gesamtmasse dieser Lösung bezogenen Massenanteil an primärem Amin auf einen Wert im Bereich von 25 % bis 50 %, insbesondere im Bereich von 30 % bis 45 %, einzustellen.

**Schritt II)** der vorliegenden Erfindung, die Bereitstellung der für die Phosgenierung erforderlichen Phosgenlösung, kann ebenfalls nach allen aus dem Stand der Technik bekannten Verfahren erfolgen. Es sind die gleichen Mischeinrichtungen und Lösungsmittel geeignet wie zuvor für das primäre Amin beschrieben. Insbesondere ist es bevorzugt, das primäre Amin in Schritt I) und Phosgen in Schritt II) jeweils in demselben Lösungsmittel, ganz besonders bevorzugt also in MCB, zu lösen. Verfahren zur Herstellung von Phosgen sind dem Fachmann bekannt und bedürfen daher keine weitere Erläuterung an dieser Stelle.

Bevorzugt ist eine Temperatur der resultierenden Phosgen-Lösung im Bereich von -20 °C bis 120 °C, insbesondere im Bereich von -10 °C bis 30 °C. Dies kann prinzipiell erreicht werden durch entsprechende Temperierung der Ausgangsstoffe Phosgen und Lösungsmittel unter Berücksichtigung der Lösungsenthalpie. Es ist erfindungsgemäß jedoch bevorzugt, insbesondere zusätzlich zur genannten Temperierung der Ausgangsstoffe, einen der Vermischung von Phosgen und Lösungsmittel nachgeschalteten Wärmeaustauscher bereitzustellen, der die gezielte Einstellung der Phosgen-Lösung auf die gewünschte Temperatur im Bereich von -20 °C bis 120 °C, insbesondere im Bereich von -10 °C bis 30 °C, ermöglicht, der also je nachdem welche Temperatur unmittelbar nach der Vermischung der Ausgangsstoffe vorliegt, heizen oder kühlen kann. Hierzu sind die gleichen Wärmeaustauscher geeignet wie zuvor für das primäre Amin beschrieben.

Im Hinblick auf die Phosgenkonzentration in der in Schritt II) bereitgestellten Lösung ist es bevorzugt, den auf die Gesamtmasse dieser Lösung bezogenen Massenanteil an Phosgen auf einen Wert im Bereich von 45 % bis 90 %, insbesondere im Bereich von 55 % bis 80 %, einzustellen.

In **Schritt III)** des erfindungsgemäßen Verfahrens erfolgt das Vermischen der in Schritt I) bereitgestellten Lösung des primären Amins und der in Schritt II) bereitgestellten Lösung. Hierzu eignen sich dem Fachmann bekannte Mischeinrichtungen wie statische oder dynamische Mischer. Statische Mischer sind durch die Abwesenheit beweglicher Teile gekennzeichnet, insbesondere seien hier Mischrohre mit statischen Mischern als Einbauten (häufig auch kurz als *Statikmischer* bezeichnet) oder Düsen genannt. Dynamische Mischer enthalten dagegen bewegliche Teile wie beispielsweise Rührorgane. Insbesondere sind hier auch die aus EP 0 830 894 A1 und EP 2 077 150 A1 bekannten Rotor-Stator-Systeme zu nennen. Dynamische Mischer, insbesondere solche vom Rotor-Stator-Typ, sind für den Einsatz in der vorliegenden Erfindung bevorzugt.

Bevorzugt wird die Mischeinrichtung aus Schritt III) nicht beheizt und nicht gekühlt, d. h. die Temperatur des erhaltenen Reaktionsgemisches wird allein durch die Mischungsenthalpie und die Enthalpie der bereits in der Mischeinrichtung einsetzenden Reaktionen bestimmt. Eine Transportleitung für das Reaktionsgemisch zwischen dem Austritt der Mischeinrichtung aus Schritt (III) und dem Eintritt in die Reaktionszone aus Schritt (IV) wird vorzugsweise ebenfalls weder beheizt noch gekühlt, ist jedoch vorzugsweise wärmeisoliert.

Erfindungsgemäß wird bei der Vermischung in Schritt III) ein auf die Aminogruppen des primären Amins bezogener stöchiometrischer Überschuss an Phosgen im Bereich von 40 % bis 200 % der Theorie, bevorzugt im Bereich von 40 % bis 120 % der Theorie, besonders bevorzugt im Bereich von 50 % bis 100 % der Theorie, ganz besonders bevorzugt im Bereich von 50 % bis 75 % der Theorie eingehalten.

In **Schritt IV)** des erfindungsgemäßen Verfahrens findet der erste Hauptteil der Reaktion zum Isocyanat statt, und zwar unter adiabatischen Bedingungen. Damit ist gemeint, dass die Schritt IV) durchlaufende Reaktionsmischung während der Umsetzung weder geheizt noch gekühlt wird. Die verwendeten Apparaturen sind gegen Wärmeverluste isoliert, sodass die Temperaturentwicklung von der Reaktionsenthalpie der ablaufenden Umsetzungen bestimmt wird.

Ohne auf eine Theorie festgelegt sein zu wollen ist davon auszugehen, dass in Schritt IV) mehrere Umsetzungen parallel ablaufen. Das primäre Amin reagiert mit Phosgen zur bekannten Zwischenstufe Carbaminsäurechlorid (exotherme Reaktion). Der dabei freigesetzte Chlorwasserstoff reagiert mit noch nicht umgesetztem Amin zu Aminhydrochlorid (exotherme Reaktion), das sich im eingesetzten Lösungsmittel löst (endotherme Reaktion). Partiell findet auch in Schritt IV) bereits die Spaltung des Carbaminsäurechlorids zum gewünschten Isocyanat und Chlorwasserstoff statt (endotherme Reaktion). Die Temperaturänderung hängt vom Zusammenspiel aller dieser Reaktionen statt. In der Regel wird in Schritt IV) nur eine geringe Temperaturänderung beobachtet, was dafür spricht, dass sich exo- und endotherme Reaktionen "die Waage halten". In jedem Fall bildet sich bei den Umsetzungen in Schritt IV) eine Gasphase aus, die in der Trennzone von der verbleibenden Flüssigphase getrennt wird. Reaktionszone und Trennzone sind bevorzugt in einem gemeinsamen Reaktor angeordnet. Hierzu eignen sich übliche, dem Fachmann bekannte Phosgenierungsreaktoren, wie insbesondere aufrecht angeordnete röhrenförmige Reaktoren (Rohrreaktoren; ist das Verhältnis von Höhe zu Durchmesser relativ klein, spricht man auch von Turmreaktoren oder Reaktortürmen), die bevorzugt mit dem in Schritt III) erhaltenen Reaktionsgemisch von unten nach oben durchströmt werden. Zur Einengung der Verweilzeitverteilung können die Reaktoren in der Reaktionszone durch dem Fachmann bekannte Einbauten segmentiert sein. Im oberen Teil des Reaktors werden die gebildete Gasphase und die verbleibende Flüssigphase getrennt entnommen. Die Phasentrennung findet spontan statt.

In **Schritt V**) des erfindungsgemäßen Verfahrens wird das in Schritt IV) erhaltene flüssige Verfahrensprodukt in zwei oder drei Stufen auf einen geringeren Druck entspannt, und zwar bevorzugt auf einen Druck im Bereich von 1,0 bar$_{(abs.)}$ bis 20 bar$_{(abs.)}$, gemessen in der in der letzten Stufe erhaltenen Gasphase. Hierzu werden Gas-Flüssigkeits-Trennbehälter (auch als *Gasabscheider* bezeichnet) eingesetzt, die für die erfindungsgemäße Vorgehensweise in Reihe geschaltet werden. Dabei bildet sich in jeder Stufe eine Chlorwasserstoff und nicht umgesetztes Phosgen enthaltende Gasphase aus. In dieser mehrstufigen Entspannung ist die nach Entspannung der ersten Stufe erhaltene Flüssigphase der Einsatzstoff der zweiten Stufe usw.

In einer Ausführungsform der Erfindung umfasst das Entspannen in Schritt V) nur die Stufen (i) und (ii), wobei das Entspannen so durchgeführt wird, dass die erste Gasphase unter einem Druck im Bereich von 10 bar$_{(abs.)}$ bis 20 bar$_{(abs.)}$, insbesondere 12 bar$_{(abs.)}$ bis 17 *bar*$_{(abs.)}$, anfällt, und die zweite Gasphase unter einem Druck im Bereich von 1,0 bar$_{(abs.)}$ bis 5,0 bar$_{(abs.)}$, insbesondere 2,0 bar$_{(abs.)}$ bis 3,0 bar$_{(abs.)}$, anfällt.

In einer weiteren Ausführungsform der Erfindung umfasst das Entspannen in Schritt V) die Stufen (i), (ii) und (iii), wobei das Entspannen so durchgeführt wird, dass die erste Gasphase unter einem Druck im Bereich von 15 bar$_{(abs.)}$ bis 20 *bar*$_{(abs.)}$, insbesondere 17 bar$_{(abs.)}$ bis 18 bar$_{(abs.)}$, anfällt, und die zweite Gasphase unter einem Druck im Bereich von 5,0 bar$_{(abs.)}$ bis 10 bar$_{(abs.)}$, insbesondere 7,0 bar$_{(abs.)}$ bis 8,0 *bar*$_{(abs.)}$, anfällt, und die dritte Gasphase unter einem Druck im Bereich von 1,0 bar$_{(abs.)}$ bis 5,0 bar$_{(abs.)}$, insbesondere 2,0 bar$_{(abs.)}$ bis 3,0 bar$_{(abs.)}$, anfällt.

In jeder dieser Ausführungsformen ist es bevorzugt, den für die letzte Entspannung vorgesehenen Gas-Flüssigkeits-Trennbehälter und die indirekt beheizte Reaktionszone aus Schritt VI) in einem gemeinsamen Apparat anzuordnen. Ein Beispiel für eine mögliche Ausgestaltung wird unten beschrieben.

In **Schritt VI**) wird die in Schritt IV) nach der letzten Entspannungsstufe verbleibende Flüssigphase in einer indirekt beheizten Reaktionszone unter Ausbildung einer Chlorwasserstoffund Phosgen-haltigen Gasphase weiter umgesetzt ("isotherme Verfahrensführung"). Dies kann in dem Fachmann bekannten heizbaren Reaktoren stattfinden. Insbesondere geeignet sind zu diesem Zweck (vertikal angeordnete) Rohrbündelreaktoren. Dabei kann die Flüssigphase aus Schritt IV) durch das Innere der Rohre des Rohrbündelreaktors (Rohrinnenraum) oder durch den Raum zwischen den Rohren des Rohrbündelreaktors, der nach außen durch die das Rohrbündel umhüllende Reaktorwand begrenzt wird (Rohraußenraum), geführt werden. Das Heizmedium - ein Wärmeträgeröl, eine Salzschmelze, Dampf oder dergleichen - wird dann durch den jeweils anderen Raum geführt, sodass es nicht in stofflichen Kontakt mit dem umzusetzenden flüssigen Verfahrensprodukt tritt (indirekte Beheizung). Die Flüssigphase aus der letzten Entspannungsstufe von Schritt IV) durchläuft dabei den vertikal angeordneten Rohrbündelreaktor vorzugsweise von oben nach unten. In der bevorzugten Ausgestaltung der Erfindung unter Integration des für die letzte Entspannungsstufe von Schritt IV) vorgesehenen Gas-Flüssigkeits-Trennbehälters in den die indirekt beheizte Reaktionszone enthaltenden Apparat aus Schritt V) findet dann die Gas-Flüssig-Phasentrennung in einer Haube am Kopf des Rohrbündelreaktors statt.

In den Schritten IV), V) und VI) fallen Gasphasen enthaltend Chlorwasserstoff und Phosgen sowie gegebenenfalls Lösungsmittel an. Bevorzugt werden diese Gasphasen zur Rückgewinnung von Wertprodukten aufgearbeitet. Die Aufarbeitung dient insbesondere der Trennung von Phosgen und Chlorwasserstoff voneinander und von Verunreinigungen und kann beispielsweise durch Absorption des Phosgens in einem Lösungsmittel oder durch destillative Trennung nach Kompression und Verflüssigung erfolgen. Das erhaltene Chlorwasserstoffgas eignet sich für die weitere Oxidation zu Chlor, welches für die Herstellung des für Schritt II) erforderlichen Phosgens benötigt wird. Die Oxidation kann elektrolytisch oder katalytisch mit Sauerstoff (sog. Deacon-Prozess) erfolgen. Zurückgewonnenes Phosgen, gegebenenfalls Lösungsmittel enthaltend, kann in Schritt II) verwendet werden.

In jedem Fall ist es zweckmäßig, die in den Schritten IV), V) und VI) anfallenden Gasphasen vor der Aufarbeitung auf einen gemeinsamen Druck einzustellen und zu vereinigen.

Grundsätzlich kann dies geschehen, in dem alle Gasphasen auf den geringsten Druck (d. h. auf den Druck der in Schritt VI) erhaltenen Gasphase) oder einen noch geringeren Druck entspannt und dann weiter aufgereinigt werden. Diese Vorgehensweise ist insbesondere bei Aufarbeitung durch Absorption bevorzugt.

Die Tatsache, dass im erfindungsgemäßen Verfahren ein Großteil der Chlorwasserstoff- und Phosgen-haltigen Gasphasen bei vergleichsweise hohem Druck anfällt, eröffnet jedoch Vorteile insbesondere bei der Reinigung durch Destillation. Bevorzugt ist dabei eine Vorgehensweise, bei welcher die in Schritt V) erhaltene zweite Gasphase und die gegebenenfalls erhaltene dritte Gasphase durch Kompression auf den Druck der ersten Gasphase eingestellt und die erste, zweite und gegebenenfalls dritte Gasphase anschließend miteinander sowie nach entsprechender Druckanpassung mit den Gasphasen aus Schritt IV) und Schritt VI) vereinigt werden. Die in Schritt IV) erhaltene Gasphase wird dabei auf den Druck der ersten Gasphase von Schritt V) entspannt. Die in Schritt VI) erhaltene Gasphase steht unter einem geringeren Druck als die erste Gasphase von Schritt V) und wird auf diesen Druck komprimiert. Wird Schritt V) dagegen einstufig durchgeführt, so fällt *die gesamte* bei der Entspannung entstehende Gasphase bei einem relativ geringen Druck an (nämlich bei einem Druck, der dem Druck der in der letzten Entspannungsstufe des erfindungsgemäßen Verfahrens erhaltenen Gasphase entspricht) und muss folglich auch *in ihrer Gesamtheit* komprimiert werden. Insgesamt ist bei erfindungsgemäßer Vorgehensweise die Menge der *zu komprimierenden* Gasströme vergleichsweise gering, was im Vergleich zu einer einstufigen Entspannung die Möglichkeit des Einsatzes kleinerer Kompressoren ermöglicht, wodurch die Investitionskosten und der Wartungsaufwand verringert werden. Die Einstellung aller Gasphasen auf den (vergleichsweise hohen) Druck der ersten Gasphase aus Schritt V) (anstelle auf den geringeren Druck von beispielsweise der letzten Gasphase aus Schritt V) oder der Gasphase aus Schritt VI)) vereinfacht in der weiteren Aufarbeitung die Kondensation der vereinigten Gasphasen, die erforderlich ist, um Chlorwasserstoff und Phosgen destillativ zu trennen. In dieser Ausführungsform schließt sich daher an die Vereinigung der auf den gemeinsamen Druck eingestellten Gasphasen vorzugsweise deren Verflüssigung durch Kondensation und Kompression und anschließende Destillation an. In dieser Destillation werden gereinigtes Chlorwasserstoffgas als Kopfprodukt und flüssiges Phosgen (oder ein Phosgen-Lösungsmittel-Gemisch) als Sumpfprodukt, das in den Prozess rezykliert werden kann, erhalten. Die Kombination des erfindungsgemäßen Verfahrens mit der katalytischen Oxidation des Chlorwasserstoffs mit Sauerstoff - ein Verfahren, das vergleichsweise hohe Anforderungen an die Reinheit der Reaktanden stellt - ist besonders vorteilhaft, da im erfindungsgemäßen Verfahren ein Großteil der Chlorwasserstoff- und Phosgen-haltigen Gasphasen verfahrensimmanent bei vergleichsweise hohem Druck anfällt, was, wie oben erwähnt, die zur destillativen Reinigung erforderliche Kompression erleichtert. Außerdem wird es dadurch wirtschaftlich, die Destillation des Phosgen-Chlorwasserstoff-Gemisches bei vergleichsweise hohem Druck durchzuführen, was für einen Deacon-Prozess, bei dem der zugeführte Chlorwasserstoff unter erhöhtem Druck steht, vorteilhaft ist.

Durch das erfindungsgemäße Verfahren ergeben sich mindestens die folgenden Vorteile ohne die Produktqualität hinsichtlich Nebenkomponentenbildung, Farbe, Acidität, NCO-Gehalt und Eisengehalt zu beeinträchtigen:

    i) Vermeidung von Aufschäumen der Reaktionslösung im Schritt der Druckerniedrigung; dadurch Sicherstellung einer stabilen Betriebsweise;
    ii) Nutzung des höheren Gasdruckes für eine anschließende destillative Trennung des Phosgen-HCl Gasgemisches zur Wiederverwendung des Phosgens in Schritt II) und Erzeugung eines phosgenfreien HCl-Gases zur weiteren Gewinnung von $Cl_2$-Gas (Einsatzstoff zur Phosgenherstellung);
    iii) Möglichkeit der Verwendung kleinerer Kompressoren, dadurch verringerter Investitions- und Wartungsaufwand sowie reduzierter Platzbedarf.

Nachfolgend wird die Erfindung anhand von Beispielen näher erläutert.

**Beispiele:**

**Beispiel 1 (erfindungsgemäß): Computer-Simulation des erfindungsgemäßen Verfahrens für die Produktion im industriellen Maßstab, zweistufige Entspannung.**

In einer MDI-Anlage werden 40,0 t/h MDA mit einer Temperatur von 130 °C mit 94,5 t/h MCB einer Temperatur von 52 °C als Lösungsmittel über einen statischen Mischer zu einer 30,0%igen MDA-Lösung vermischt (**Schritt I**)). Phosgen wird in einem Phosgenlösungstank mit MCB unter Erhalt einer 60,0%igen Phosgenlösung vermischt (**Schritt II**)). Stündlich werden 106,7 Tonnen dieser Phosgenlösung einer Temperatur von 3,0 °C durch einen Wärmeaustauscher geführt und so auf eine Temperatur von -1 °C abgekühlt. Analog werden stündlich 134,5 Tonnen der 30,0%igen MDA-Lösung einer Temperatur von 80,0 °C durch einen Wärmeaustauscher geführt und so auf eine Temperatur von 60 °C abgekühlt. Die so temperierten MDA- und Phosgenlösungen werden in einen dynamischen Mischer geführt (**Schritt III**)). Die Temperatur am Austritt des dynamischen Mischers wird auf 130 °C eingestellt.

Die den Mischer verlassende flüssige Reaktionsmischung wird unter adiabatischen Bedingungen durch einen gegen Wärmeverluste isolierten Phosgenierreaktor (Turmreaktor) geführt (**Schritt IV**)). Der Druck am Austritt des Phosgenierreaktors wird mittels eines Druckhalteventils auf 22 bar$_{(abs.)}$ eingestellt; die Austrittstemperatur beträgt 120 °C. Die Verweilzeit der Phosgenierungsreaktion vom Mischer bis zum Austritt aus dem Phosgenierreaktors beträgt 5 min. Im Kopf des Turmreaktors scheidet sich eine HCl- und Phosgen-haltige Gasphase (die auch noch Anteile an verdampftem MCB enthält) ab. Die dem Reaktor entnommene Reaktionslösung wird in zwei Stufen in jeweils einem Gasabscheider auf zunächst 15 bar$_{(abs.)}$ und dann 3 bar$_{(abs.)}$ entspannt (**Schritt V**)) und anschließend in einem beheizten Reaktor bei 130 °C und 3 bar$_{(abs.)}$ weiter umgesetzt (**Schritt VI**)).

Anschließend wird die den beheizten Reaktor verlassende Reaktionslösung unter Rückgewinnung des Lösungsmittels und Erhalt des Isocyanats aufgearbeitet (**Schritt VII**)). Die Aufarbeitung umfasst eine Entphosgenierung und Lösungsmittelabtrennung. In der Destillation zur Lösungsmittelabtrennung fallen als Sumpfprodukt 50,0 t/h MDI an, das mittels weiterer Destillationsschritte in Methylen-Diphenylen-Diisoyanat und ein Gemisch aus Methylen-Diphenylen-Diisoyanat und Polymethylen-Polyphenylen-Polyisocyanat getrennt wird.

In einer realen Produktionsanlage können die hier genannten Mengenströme vorteilhafterweise in mehreren parallel betriebenen Reaktionsstraßen umgesetzt werden.

## Patentansprüche

1. Verfahren zur Herstellung eines Isocyanats durch Umsetzung eines primären Amins mit Phosgen, umfassend die Schritte:

   I) Bereitstellen einer Lösung des primären Amins in einem Lösungsmittel;
   II) Bereitstellen einer Lösung von Phosgen in einem Lösungsmittel;
   III) Vermischen der in Schritt I) bereitgestellten Lösung des primären Amins und der in Schritt II) bereitgestellten Lösung von Phosgen in einer Mischeinrichtung zu einem Reaktionsgemisch einer Temperatur im Bereich von 110 °C bis 145 °C unter Einhaltung eines auf die Aminogruppen des primären Amins bezogenen stöchiometrischen Überschusses an Phosgen im Bereich von 40 % bis 200 % der Theorie;
   IV) Führen des in Schritt III) erhaltenen Reaktionsgemisches durch eine Reaktionszone und durch eine dieser Reaktionszone strömungstechnisch nachgeschaltete Trennzone unter Ausbildung einer unter einem Druck im Bereich von 8,0 bar$_{(abs.)}$ bis 50,0 bar$_{(abs.)}$ stehenden Gasphase aus dem flüssigen Reaktionsgemisch in der Trennzone, wobei die Reaktionszone und die Trennzone nicht beheizt und nicht gekühlt werden, wobei die in der Trennzone gebildete Gasphase und die verbleibende Flüssigphase der Trennzone getrennt voneinander entnommen werden;
V) Entspannen der der Trennzone aus Schritt IV) entnommenen Flüssigphase unter partieller Überführung dieser Flüssigphase in die Gasphase;
VI) Führen der in Schritt V) nach dem Entspannen verbleibenden Flüssigphase durch eine indirekt beheizte Reaktionszone, wobei sich eine Chlorwasserstoff- und Phosgen-haltige Gasphase ausbildet, die abgetrennt wird, und eine Isocyanat- und Lösungsmittel enthaltende Flüssigphase verbleibt, die der indirekt beheizten Reaktionszone entnommen wird;
VII) Aufarbeiten der in Schritt VI) gewonnenen Isocyanat- und Lösungsmittel enthaltenden Flüssigphase unter Rückgewinnung des Lösungsmittels und Erhalt des Isocyanats;

**dadurch gekennzeichnet, dass**
in Schritt V) zum Entspannen der der Trennzone aus Schritt IV) entnommenen Flüssigphase diese Flüssigphase

   (i) zunächst in einem ersten Gas-Flüssigkeits-Trennbehälter entspannt wird, wobei eine erste Flüssigphase und eine erste Gasphase gebildet werden,
   (ii) woran anschließend die erste Flüssigphase in einem zweiten Gas-Flüssigkeits-Trennbehälter weiter entspannt wird, wobei eine zweite Flüssigphase und eine zweite Gasphase gebildet werden,
   (iii) woran anschließend optional die zweite Flüssigphase in einem dritten Gas-Flüssigkeits-Trennbehälter weiter entspannt wird, wobei eine dritte Flüssigphase und eine dritte Gasphase gebildet werden,

wobei die in Schritt VI) durch die indirekt beheizte Reaktionszone geführte Flüssigphase die zweite oder dritte Flüssigphase ist.

2. Verfahren gemäß Anspruch 1, bei welchem die in Schritt I) bereitgestellte Lösung des primären Amins einen auf die Gesamtmasse dieser Lösung bezogenen Massenanteil an primärem Amin im Bereich von 25 % bis 50 % hat und die in Schritt II) bereitgestellte Lösung von Phosgen einen auf die Gesamtmasse dieser Lösung bezogenen Massenanteil an Phosgen im Bereich von 45 % bis 90 % hat.

3. Verfahren gemäß einem der vorstehenden Ansprüche, bei welchem die Mischeinrichtung aus Schritt III) nicht beheizt und nicht gekühlt wird.

4. Verfahren gemäß einem der vorstehenden Ansprüche, bei welchem die in Schritt III) eingesetzte Mischeinrichtung einen oder mehrere dynamischen Mischer umfasst.

5. Verfahren gemäß einem der vorstehenden Ansprüche, bei welchem Reaktionszone und Trennzone aus Schritt IV) in einem gemeinsamen Reaktor angeordnet sind.

6. Verfahren gemäß Anspruch 5, bei welchem der Reaktor ein aufrecht angeordneter röhrenförmiger Reaktor ist.

7. Verfahren gemäß Anspruch 6, bei welchem der Reaktor mit dem in Schritt III) erhaltenen Reaktionsgemisch von unten nach oben durchströmt wird.

8. Verfahren gemäß einem der vorstehenden Ansprüche, bei welchem die indirekt beheizte Reaktionszone aus Schritt VI) Bestandteil eines Rohrbündelreaktors ist, durch dessen Rohrinnenraum die in Schritt V) nach dem Entspannen verbleibende Flüssigphase geführt wird und durch dessen Rohraußenraum ein Heizmedium geführt wird, oder durch dessen Rohraußenraum die in Schritt V) nach dem Entspannen verbleibende Flüssigphase geführt wird und durch dessen Rohrinnenraum ein Heizmedium geführt wird.

9. Verfahren gemäß einem der vorstehenden Ansprüche, bei welchem das Entspannen in Schritt V) nur die Stufen (i) und (ii) umfasst, wobei das Entspannen so durchgeführt wird, dass die erste Gasphase unter einem Druck im Bereich von 10 bar$_{(abs.)}$ bis 20 bar$_{(abs.)}$ anfällt, und die zweite Gasphase unter einem Druck im Bereich von 1,0 bar$_{(abs.)}$ bis 5,0 bar$_{(abs.)}$ anfällt.

10. Verfahren gemäß einem der Ansprüche 1 bis 8, bei welchem das Entspannen in Schritt V) die Stufen (i), (ii) und (iii) umfasst, wobei das Entspannen so durchgeführt wird, dass die erste Gasphase unter einem Druck im Bereich von 15 bar$_{(abs.)}$ bis 20 bar$_{(abs.)}$ anfällt, und die zweite Gasphase unter einem Druck im Bereich von 5,0 bar$_{(abs.)}$ bis 10 bar$_{(abs.)}$ anfällt, und die dritte Gasphase unter einem Druck im Bereich von 1,0 bar$_{(abs.)}$ bis 5,0 bar$_{(abs.)}$ anfällt.

11. Verfahren gemäß einem der vorstehenden Ansprüche, bei welchem die in den Schritten IV), V) und VI) anfallenden Gasphasen zur Gewinnung von Chlorwasserstoff und Phosgen sowie gegebenenfalls Lösungsmittel aufgearbeitet werden.

12. Verfahren gemäß Anspruch 11, bei welchem die in den Schritten IV), V) und VI) anfallenden Gasphasen vor der Aufarbeitung auf einen gemeinsamen Druck eingestellt und vereinigt werden.

13. Verfahren gemäß Anspruch 12, bei welchem die in Schritt IV) erhaltene Gasphase, die in Schritt V) erhaltene zweite Gasphase und die gegebenenfalls erhaltene dritte Gasphase, sowie die in Schritt VI) erhaltene Gasphase auf den Druck der ersten in Schritt V) erhaltenen Gasphase eingestellt werden.

14. Verfahren gemäß Anspruch 13, bei welchem die vereinigten Gasphasen kondensiert und komprimiert und zur Trennung von Chlorwasserstoff und Phosgen destilliert werden.

15. Verfahren gemäß einem der vorstehenden Ansprüche, bei welchem

    (i) Methylen-Diphenylen-Diisocyanat und/oder Polymethylen-Polyphenylen-Polyisocyanat durch Umsetzung von Methylen-Diphenylen-Diamin und/oder Polymethylen-Polyphenylen-Polyamin mit Phosgen oder
    (ii) Toluylendiisocyanat durch Umsetzung von Toluylendiamin mit Phosgen hergestellt wird.

**Claims**

1. Process for preparing an isocyanate by reacting a primary amine with phosgene, comprising the steps of:

    I) providing a solution of the primary amine in a solvent;
    II) providing a solution of phosgene in a solvent;
    III) mixing the solution of the primary amine provided in step I) and the solution of phosgene provided in step II) in a mixing unit to give a reaction mixture of a temperature in the range from 110°C to 145°C with observance of a stoichiometric excess of phosgene based on the amino groups of the primary amine in the range from 40% to 200% of theory;
    IV) running the reaction mixture obtained in step III) through a reaction zone and through a separation zone downstream in flow direction of this reaction zone to form a gas phase under a pressure in the range from 8.0 bar$_{(abs.)}$ to 50.0 bar$_{(abs.)}$ from the liquid reaction mixture in the separation zone, where the reaction zone and the separation zone are not heated and not cooled, where the gas phase formed in the separation zone and the remaining liquid phase are removed from the separation zone separately from one another;

V) expanding the liquid phase withdrawn from the separation zone from step IV) with partial conversion of this liquid phase to the gas phase;

VI) running the liquid phase that remains after the expansion in step V) through an indirectly heated reaction zone, thereby forming a hydrogen chloride- and phosgene-containing gas phase which is removed, and whereby an isocyanate- and solvent-containing liquid phase remains which is withdrawn from the indirectly heated reaction zone;

VII) working up the isocyanate- and solvent-containing liquid phase obtained in step VI) to recover the solvent and obtain the isocyanate;

**characterized in that**

in step V) the liquid phase withdrawn from the separation zone from step IV) is expanded by

(i) first expanding this liquid phase in a first gas-liquid separation vessel, forming a first liquid phase and a first gas phase,

(ii) then subsequently further expanding the first liquid phase in a second gas-liquid separation vessel, forming a second liquid phase and a second gas phase,

(iii) then subsequently optionally further expanding the second liquid phase in a third gas-liquid separation vessel, forming a third liquid phase and a third gas phase,

where the liquid phase run through the indirectly heated reaction zone in step VI) is the second or the third liquid phase.

2. Process according to Claim 1, in which the solution of the primary amine provided in step I) has a proportion by mass of primary amine based on the total mass of this solution in the range from 25% to 50% and the solution of phosgene provided in step II) has a proportion by mass of phosgene based on the total mass of this solution in the range from 45% to 90%.

3. Process according to any of the preceding claims, in which the mixing unit from step III) is not heated and not cooled.

4. Process according to any of the preceding claims, in which the mixing unit used in step III) comprises one or more dynamic mixers.

5. Process according to any of the preceding claims, in which reaction zone and separation zone from step IV) are arranged in a common reactor.

6. Process according to Claim 5, in which the reactor is a tubular reactor in an upright arrangement.

7. Process according to Claim 6, in which the reaction mixture obtained in step III) flows through the reactor from the bottom upward.

8. Process according to any of the preceding claims, in which the indirectly heated reaction zone from step VI) is part of a shell and tube reactor, the liquid phase remaining after the expansion in step V) is run through the tube interior thereof and a heating medium is run through the tube exterior thereof, or the liquid phase remaining after the expansion in step V) is run through the tube exterior thereof and a heating medium is run through the tube interior thereof.

9. Process according to any of the preceding claims, in which the expanding in step V) comprises stages (i) and (ii) only, where the expanding is conducted in such a way that the first gas phase is obtained under a pressure in the range from 10 $bar_{(abs.)}$ to 20 $bar_{(abs.)}$, and the second gas phase is obtained under a pressure in the range from 1.0 $bar_{(abs.)}$ to 5.0 $bar_{(abs.)}$.

10. Process according to any of Claims 1 to 8, in which the expanding in step V) comprises stages (i), (ii) and (iii), where the expanding is conducted in such a way that the first gas phase is obtained under a pressure in the range from 15 $bar_{(abs.)}$ to 20 $bar_{(abs.)}$, and the second gas phase is obtained under a pressure in the range from 5.0 $bar_{(abs.)}$ to 10 $bar_{(abs.)}$, and the third gas phase is obtained under a pressure in the range from 1.0 $bar_{(abs.)}$ to 5.0 $bar_{(abs.)}$.

11. Process according to any of the preceding claims, in which the gas phases obtained in steps IV), V) and VI) are worked up to obtain hydrogen chloride and phosgene and optionally solvent.

12. Process according to Claim 11, in which the gas phases obtained in steps IV), V) and VI), prior to the workup, are adjusted to a common pressure and combined.

13. Process according to Claim 12, in which the gas phase obtained in step IV), the second gas phase obtained in step V) and any third gas phase obtained, and the gas phase obtained in step VI) are adjusted to the pressure of the first gas phase obtained in step V).

14. Process according to Claim 13, in which the combined gas phases are condensed and compressed and distilled for separation of hydrogen chloride and phosgene.

**15.** Process according to any of the preceding claims, in which

(i) methylene diphenylene diisocyanate and/or polymethylene polyphenylene polyisocyanate is prepared by reacting methylene diphenylene diamine and/or polymethylene polyphenylene polyamine with phosgene or
(ii) tolylene diisocyanate is prepared by reacting tolylenediamine with phosgene.

**Revendications**

**1.** Procédé pour la préparation d'un isocyanate par transformation d'une amine primaire avec du phosgène, comprenant les étapes :

I) préparation d'une solution de l'amine primaire dans un solvant ;
II) préparation d'une solution de phosgène dans un solvant ;
III) mélange de la solution de l'amine primaire préparée dans l'étape I) et de la solution de phosgène préparée dans l'étape II) dans un dispositif de mélange en un mélange réactionnel d'une température dans la plage de 110°C à 145°C avec respect d'un excès stœchiométrique de phosgène par rapport aux groupes amino de l'amine primaire dans la plage de 40% à 200% de la théorie ;
IV) guidage du mélange réactionnel obtenu dans l'étape III) à travers une zone de réaction et à travers une zone de séparation disposée en aval, d'un point de vue technique d'écoulement, de cette zone de réaction avec formation d'une phase gazeuse se trouvant à une pression dans la plage de 8,0 bars$_{(abs.)}$ à 50,0 bars$_{(abs.)}$ à partir du mélange réactionnel liquide dans la zone de séparation, la zone de réaction et la zone de séparation n'étant ni chauffées ni refroidies, la phase gazeuse formée dans la zone de séparation et la phase liquide résiduelle étant prélevées séparément l'une de l'autre de la zone de séparation ;
V) détente de la phase liquide prélevée de la zone de séparation de l'étape IV) avec transfert partiel de cette phase liquide en phase gazeuse ;
VI) guidage de la phase liquide résiduelle après la détente dans l'étape V) à travers une zone de réaction chauffée de manière indirecte, une phase gazeuse contenant de l'acide chlorhydrique et du phosgène se formant, qui est séparée, et une phase liquide contenant de l'isocyanate et du solvant restant, qui est prélevée de la zone de réaction chauffée de manière indirecte ;
VII) traitement de la phase liquide contenant de l'isocyanate et du solvant obtenue dans l'étape VI) avec récupération du solvant et obtention de l'isocyanate ;

**caractérisé en ce que** dans l'étape V), pour détendre la phase liquide prélevée de la zone de séparation de l'étape IV), cette phase liquide

( i) est d'abord détendue dans un premier récipient de séparation gaz-liquide, une première phase liquide et une première phase gazeuse étant formées,
( ii) la première phase liquide étant ensuite détendue davantage dans un deuxième récipient de séparation de gaz-liquide, une deuxième phase liquide et une deuxième phase gazeuse étant formées,
( iii) la deuxième phase liquide étant ensuite éventuellement détendue davantage dans un troisième récipient de séparation gaz-liquide, une troisième phase liquide et une troisième phase gazeuse étant formées,

la phase liquide guidée à travers la zone de réaction chauffée de manière indirecte dans l'étape VI) étant la deuxième ou la troisième phase liquide.

**2.** Procédé selon la revendication 1, dans lequel la solution de l'amine primaire préparée dans l'étape I) présente une proportion massique d'aminé primaire, par rapport à la masse totale de cette solution, dans la plage de 25% à 50% et la solution de phosgène préparée dans l'étape II) présente une proportion massique de phosgène, par rapport à la masse totale de cette solution, dans la plage de 45 à 90%.

**3.** Procédé selon l'une quelconque des revendications précédentes, dans lequel le dispositif de mélange de l'étape III) n'est ni chauffé ni refroidi.

**4.** Procédé selon l'une quelconque des revendications précédentes, dans lequel le dispositif de mélange utilisé dans l'étape III) comprend un ou plusieurs mélangeurs dynamiques.

**5.** Procédé selon l'une quelconque des revendications précédentes, dans lequel la zone de réaction et la zone de séparation de l'étape IV) sont disposées dans un réacteur commun.

**6.** Procédé selon la revendication 5, dans lequel le réacteur est un réacteur tubulaire disposé verticalement.

**7.** Procédé selon la revendication 6, dans lequel le réacteur est traversé de bas en haut par le mélange réactionnel obtenu dans l'étape III).

8. Procédé selon l'une quelconque des revendications précédentes, dans lequel la zone de réaction chauffée de manière indirecte de l'étape VI) fait partie d'un réacteur à faisceau tubulaire à travers l'espace interne tubulaire duquel la phase liquide restant après la détente dans l'étape V) est guidée et à travers l'espace externe tubulaire duquel un milieu chauffant est guidé ou à travers l'espace externe tubulaire duquel la phase liquide restant après la détente dans l'étape V) est guidée et à travers l'espace interne tubulaire duquel un milieu chauffant est guidé.

9. Procédé selon l'une quelconque des revendications précédentes, dans lequel la détente dans l'étape V) ne comprend que les étages (i) et (ii), la détente étant réalisée de manière telle que la première phase gazeuse est obtenue à une pression dans la plage de 10 bars$_{(abs.)}$ à 20 bars$_{(abs.)}$ et la deuxième phase gazeuse est obtenue à une pression dans la plage de 1,0 bar$_{(abs.)}$ à 5,0 bars$_{(abs.)}$.

10. Procédé selon l'une quelconque des revendications 1 à 8, dans lequel la détente dans l'étape V) comprend les étages (i), (ii) et (iii), la détente étant réalisée de manière telle que la première phase gazeuse est obtenue à une pression dans la plage de 15 bars$_{(abs.)}$ à 20 bars$_{(abs.)}$ et la deuxième phase gazeuse est obtenue à une pression dans la plage de 5,0 bars$_{(abs.)}$ à 10,0 bars$_{(abs.)}$ et la troisième phase gazeuse est obtenue à une pression dans la plage de 1,0 bar$_{(abs.)}$ à 5,0 bars$_{(abs.)}$.

11. Procédé selon l'une quelconque des revendications précédentes, dans lequel les phases gazeuses générées dans les étapes IV), V) et VI) sont traitées pour la production d'acide chlorhydrique et de phosgène ainsi que le cas échéant de solvant.

12. Procédé selon la revendication 11, dans lequel les phases gazeuses générées dans les étapes IV), V) et VI) sont réglées à une pression commune et rassemblées avant le traitement.

13. Procédé selon la revendication 12, dans lequel la phase gazeuse obtenue dans l'étape IV), la deuxième phase gazeuse et l'éventuelle troisième phase obtenues dans l'étape V) ainsi que la phase gazeuse obtenue dans l'étape VI) sont réglées à la pression de la première phase gazeuse obtenue dans l'étape V).

14. Procédé selon la revendication 13, dans lequel les phases gazeuses rassemblées sont condensées et comprimées puis distillées pour la séparation de l'acide chlorhydrique et du phosgène.

15. Procédé selon l'une quelconque des revendications précédentes, dans lequel on prépare

(i) du diisocyanate de méthylène-diphénylène et/ou du polyisocyanate de polyméthylène-polyphénylène par transformation de méthylène-diphénylène-diamine et/ou de polyméthylène-polyphénylène-polyamine avec du phosgène ou (ii) du diisocyanate de toluylène par transformation de toluylènediamine avec du phosgène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3403204 A
- DE 1768439 A
- DE 10222968 A
- EP 1873142 A1
- EP 1616857 A1
- WO 2017001320 A1
- EP 1854783 A2
- EP 0830894 A1
- EP 2077150 A1